# EUROPEAN PATENT SPECIFICATION

(11) **EP 3 307 171 B1**
(45) Date of publication and mention of the grant of the patent: **11.08.2021**
(21) Application number: 16729232.5
(22) Date of filing: 07.06.2016
(51) Int. Cl.: A61B 8/00

(54) **ULTRASOUND IMAGING APPARATUS**
ULTRASCHALLBILDGEBUNGSVORRICHTUNG
APPAREIL D'IMAGERIE À ULTRASONS

(30) Priority: 10.06.2015 EP 15171400
(43) Date of publication of application: 18.04.2018
(73) Proprietor: Koninklijke Philips N.V., 5656 AG Eindhoven (NL)
(72) Inventor: WAECHTER-STEHLE, Irina, 5656 AE Eindhoven (NL); WEBER, Frank Michael, 5656 AE Eindhoven (NL); BUERGER, Christian, 5656 AE Eindhoven (NL)
(74) Representative: Philips Intellectual Property & Standards
(86) International application number: PCT/EP2016/062922
(87) International publication number: WO 2016/198413

(56) References cited:
- WO-A1-2013/014647
- WO-A1-2014/097090
- US-A1- 2010 210 947
- US-A1- 2013 204 134
- US-A1- 2013 253 325
- US-A1- 2014 058 263

## Description

### FIELD OF THE INVENTION

The present invention relates to an ultrasound imaging apparatus for determining at least one physical parameter associated to an anatomical object of a patient.

The present invention further relates to an ultrasound imaging method for determining at least one physical parameter associated to an anatomical object of the patient and relates to a computer program comprising program code means for causing a computer to carry out the steps of the method according to the invention when said computer program is carried out on a computer.

### BACKGROUND OF THE INVENTION

In the field of ultrasound imaging systems for medical detection, it is generally known to detect anatomical objects from different viewing directions in order to improve the image quality and to provide a non-obstructed view of the anatomical object. A corresponding ultrasound imaging system is e.g. known from WO 2014/207621 A1.

In the field of ultrasound systems, in particular for transthoracic echocardiography (TTE), ultrasound probes are currently under development, which comprise multi-probe arrays also known as large area transthoracic echocardiography (LATTE). These multi-probe arrays comprise multiple probes in a fixed relation to each other, wherein the ultrasound images of the probes can be combined to form an overview image or displayed separately to display different parts of the respective organ from different viewing directions.

The disadvantage of the known ultrasound systems is that different anatomical structures are displayed in the ultrasound image with different image qualities due to the different orientations of the different structures and that a manual optimization of the imaging quality is cumbersome for the user.

WO 2013/014647 A1 discloses an ultrasound probe, a device and method for acquiring a blood flow signal of an artery and a system for measuring systolic blood pressure. The ultrasound probe comprises a plurality of ultrasound transducers, wherein each of the plurality of ultrasound transducers is configured to obtain an echo signal by transmitting an ultrasonic wave, receiving an ultrasonic wave and transforming the received ultrasonic wave into the echo signal. Further the ultrasound probe comprises a deriving unit configured to derive, from each received echo signal, at least one Doppler signal, the at least one Doppler signal being associated with the ultrasound transducer obtaining the echo signal and being indicative of information about Doppler shift of the echo signal and a selecting unit configured to select a Doppler signal from the derived Doppler signals according to a predefined condition, the selected Doppler signal being indicative of the at least one characteristic of the blood flow in the artery of the body.

US 2014/058263 A1 discloses an adaptive ultrasound array which uses information about contact quality with the body to weight produced ultrasound data.

US 2010/210947 A1 discloses a vascular flow sensor assembly which includes an ultrasound transducer which is attached to the body by an adhesive or mechanical substrate to create acoustic coupling between the ultrasound transducer and the body.

WO 2014/097090 A1 discloses an apparatus including an imaging probe, the apparatus being configured for dynamically arranging presentation of visual feedback for guiding manual adjustment, via the probe, of a location, and orientation, associated with the probe.

### SUMMARY OF THE INVENTION

It is therefore an object of the invention to provide an ultrasound imaging apparatus and a corresponding ultrasound imaging method which provides an ultrasound detection with an improved quality and low handling effort for the user.

According to one aspect of the present invention, an ultrasound imaging apparatus is provided, comprising:
- an ultrasound acquisition unit connected to a plurality of ultrasound probes each for providing ultrasound data suitable for ultrasound imaging of a patient in a field of view of the ultrasound probes,
- a detection unit for detecting an anatomical object of the patient in the field of view on the basis of the ultrasound data received from at least one of the ultrasound probes and for determining a spatial relationship of the anatomical object and each of the ultrasound probes,
- a selection unit coupled to the detection unit for selecting at least one of the ultrasound probes on the basis of an acquisition quality of at least one physical parameter detectable from the ultrasound data, wherein the acquisition quality is determined on the basis of the spatial relationship of the anatomical object and each of the ultrasound probes and at least one anatomical feature of the anatomical object, and
- an evaluation unit coupled to the ultrasound acquisition unit for receiving the ultrasound data from the at least one selected ultrasound probe and for determining the at least one physical parameter on the basis of the ultrasound data received from the selected ultrasound probe,
wherein the detection unit is adapted to detect (i) a fiber direction of the anatomical object, (ii) a surface of the anatomical object, or (iii) a flow direction of a fluid associated to the anatomical object, and wherein the detection unit is adapted to determine an orientation of (i) the fiber direction, (ii) the surface, or (iii) the flow direction with respect to a viewing direction of each of the ultrasound probes as the spatial relationship.

According to another aspect of the present invention, an ultrasound imaging method is provided comprising the steps of:
- acquiring ultrasound data suitable for ultrasound imaging of a patient in a field of view of a plurality of ultrasound probes,
- determining an anatomical object of the patient in the field of view on the basis of the ultrasound data received from at least one of the ultrasound probes,
- detecting (i) a fiber direction of the anatomical object, (ii) a surface of the anatomical object, or (iii) a flow direction of a fluid associated to the anatomical object;
- determining a spatial relationship of the anatomical object and each of the ultrasound probes, wherein the spatial relationship includes an orientation of (i) the fiber direction, (ii) the surface, or (iii) the flow direction with respect to a viewing direction of each of the ultrasound probes,
- selecting at least one of the ultrasound probes on the basis of an acquisition quality of at least one physical parameter detectable from the ultrasound data, wherein the acquisition quality is determined on the basis of the spatial relationship of the anatomical object and each of the ultrasound probes and at least one anatomical feature of the anatomical object, and
- determining the at least one physical parameter on the basis of the ultrasound data received from the selected ultrasound probe.

According to still another aspect of the present invention, a computer program is provided comprising program code means for causing a computer to carry out the steps of the ultrasound imaging method according to the present invention, when said computer program is carried out on a computer.

Preferred embodiments of the invention are defined in the dependent claims. It shall be understood that the claimed method has similar and/or identical preferred embodiments as the claimed apparatus and as defined in the dependent claims.

The present invention is based on the idea to acquire ultrasound data of a patient by means of a plurality of ultrasound probes and to determine a spatial relationship of an anatomical object to the ultrasound probes, wherein at least one of the ultrasound probes is selected on the basis of an acquisition quality of one physical parameter determined or estimated on the basis of the spatial relationship and to determine the physical parameter on the basis of the ultrasound data received from the selected ultrasound probe. By determining the acquisition quality on the basis of the spatial relationship, it is guaranteed that the physical parameter of the anatomical object is determined by the ultrasound probe having the best viewing direction and the best signal strength to determine the physical parameter. Hence, the best image quality can be automatically achieved by selecting the ultrasound probe having an optimal spatial relationship to the anatomical object for determining the physical parameter so that the acquisition quality can be improved without an increased handling effort for the user.

The detection unit is adapted to detect a fiber direction of the anatomical object, a surface of the anatomical object, or a flow direction of a fluid associated to the anatomical object and to determine an orientation of the fiber direction, the surface, or the flow direction with respect to a viewing direction of each of the ultrasound probes as the spatial relationship. Since the signal strength of reflected ultrasound waves depends on an angle between the propagation direction of the ultrasound waves and the surface or the fiber direction of the imaged anatomical structure, the image quality can be improved by considering the fiber direction or the surface of the anatomical object and the orientation to the viewing direction of each of the ultrasound probes.

In a preferred embodiment, the acquisition quality is relatively high for an orthogonal orientation of the surface or the fiber direction with respect to the viewing direction of the respective ultrasound probe. This is a possibility to select the ultrasound probe having the best viewing angle with respect to the determined anatomical object.

In a preferred embodiment, the ultrasound probes are fixed to each other. This is a possibility to reduce the effort for determining the spatial relationship of the anatomical object to each of the ultrasound probe, since the relative position of the ultrasound probes is fixed and available as a calculation parameter.

In a preferred embodiment, the detection unit comprises a segmentation unit for determining segmentation data of the anatomical object on the basis of the ultrasound data and for determining the spatial relationship on the basis of the segmentation data. This is a possibility to precisely determine the anatomical object and the anatomical features of the anatomical object so that the acquisition quality can be determined with high precision.

In a preferred embodiment, the segmentation unit is adapted to provide the segmentation data on the basis of a predefined segmentation model of the anatomical object. This is a possibility to further reduce the segmentation effort and to improve the segmentation, since the predefined segmentation model can be used as a basis for the segmentation data.

In a preferred embodiment, the predefined segmentation model comprises anatomical features (tissue type for example) of the anatomical object. This is a possibility to further improve the determination of the acquisition quality, since additional features like ultrasound image contrast may be known in advance for different tissues so that the determination of the acquisition quality can be improved.

In a preferred embodiment, the detection unit is adapted to determine a spatial relationship of the ultrasound probes to each other on the basis of the spatial relationship of each of the probes to the anatomical object. This is a possibility to determine the spatial relationship of the ultrasound probes to each other, if the relationship is not known or if the probes are not fixed to each other, so that different ultrasound acquisition units can be used without an increased adaption effort.

In a preferred embodiment, the physical parameter is an ultrasound image of the anatomical object. The physical parameter is in particular a reflected ultrasound signal forming a B-mode ultrasound image of the anatomical object. This is a possibility to provide an ultrasound image of the anatomical object with an improved image quality, since the ultrasound probe is selected which provides the best image quality in the respective viewing direction.

In a preferred embodiment, the ultrasound acquisition unit comprises a control unit for controlling a steering direction of the ultrasound probes on the basis of the spatial relationship to the anatomical object. This is a possibility to steer ultrasound beams originating from the different ultrasound probes to the anatomical object in order to receive detailed ultrasound data from different viewing directions with high quality.

In a further preferred embodiment, the control unit is adapted to steer the beams from the selected ultrasound probes on the basis of the spatial relationship to the anatomical object. This is a possibility to further improve the detection of the physical parameter, since the selected probes are correspondingly focused.

In a preferred embodiment, the ultrasound imaging apparatus further comprises an imaging unit for providing a compound ultrasound image on the basis of the ultrasound data received from the plurality of ultrasound probes. This is a possibility to further improve the image quality, since the image is based upon a combination of the ultrasound data of the different ultrasound probes received from different viewing directions.

In a further preferred embodiment, the image data of each of the ultrasound probes is weighted on the basis of the determined acquisition quality. This is a possibility to further improve the image quality of the compound ultrasound image, since the different ultrasound data from the different ultrasound probes are weighted so that the ultrasound data having a relatively high quality have an increased probability to be used for the combined image than the ultrasound data having a relatively lower (reduced) quality.

The detection unit is adapted to determine a flow direction of a fluid associated to the anatomical object, wherein the detection unit is adapted to determine an orientation of the flow direction with respect to a viewing direction of each of the ultrasound probes as the spatial relationship. This is a possibility to determine the motion of the fluid at or in the anatomical object with an improved precision.

In a preferred embodiment, the physical parameter is a flow parameter of the fluid. This is a possibility to precisely determine the flow of the fluid in or at the anatomical object.

In a further preferred embodiment, the acquisition quality is relatively high for a parallel orientation of the flow direction with respect to a viewing direction of the respective ultrasound probe. This is a possibility to select the ultrasound probe having the best viewing direction in order to determine the motion of the fluid in or at the anatomical object, so that the motion of the fluid can be determined with high precision. It is in particular desirable to utilize a Doppler signal to determine the motion of the fluid at or in the anatomical object.

In a further preferred embodiment, the physical parameter is an anatomical structure adjacent to the anatomical object, wherein the acquisition quality is a degree of blockage of the anatomical structure by the anatomical object and wherein the evaluation unit is adapted to provide ultrasound image data of the anatomical structure received from the selected ultrasound probe. This is a possibility to provide image data of the anatomical structure adjacent to an ultrasound blocking anatomical object, such as a bone, with high quality and low handling effort for the user, since the ultrasound probe having the best viewing direction to the anatomical structure can be automatically selected.

As mentioned above, the selection of at least one of the ultrasound probes of the ultrasound acquisition unit is performed on the basis of a determined acquisition quality of the at least one physical parameter which is detectable from the ultrasound data, wherein the acquisition quality is determined or estimated based on the viewing direction of the ultrasound probe to the anatomical object and, in particular, on the basis of at least one anatomical feature of the anatomical object. The acquisition quality can be determined since the viewing direction of the ultrasound probe and the anatomical features of the anatomical object are highly relevant for the signal strength in general, so that the determination of the physical parameter on the basis of the ultrasound data can be significantly improved.

Since the selection of the ultrasound probes can be performed automatically on the basis of the determined spatial relationship, the effort to improve the ultrasound acquisition quality is not increased for the operator.

### BRIEF DESCRIPTION OF THE DRAWINGS

These and other aspects of the invention will be apparent from and elucidated with reference to the embodiments) described hereinafter. In the following drawings
Fig. 1 shows a schematic representation of an ultrasound imaging apparatus in use to scan a volume of a patient's body;
Figs. 2a, b show schematic illustrations of a viewing angle of an ultrasound probe;
Figs. 3a-f show schematic illustrations of an anatomical object analyzed from different viewing directions of different ultrasound probes; and
Fig. 4 shows a schematic illustration of a plurality of ultrasound probes for Doppler signal acquisition from different viewing directions.-

### DETAILED DESCRIPTION OF THE EMBODIMENTS

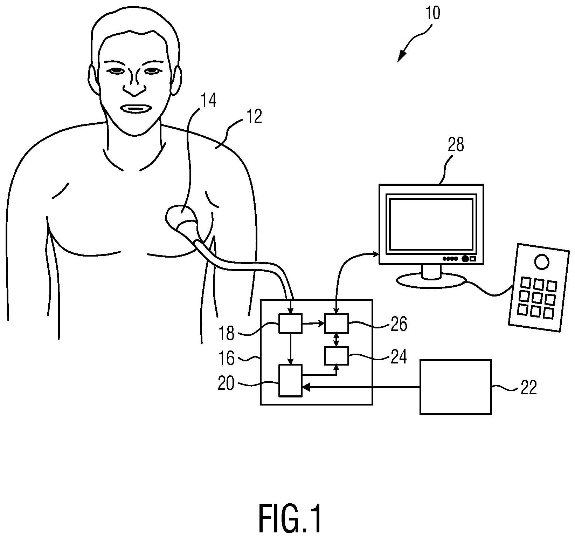

Fig. 1 shows a schematic illustration of an ultrasound imaging apparatus generally denoted by 10. The ultrasound imaging apparatus 10 is applied to inspect a volume of an anatomical site, in particular an anatomical site of a patient 12. The ultrasound imaging apparatus 10 comprises an ultrasound acquisition unit 14 comprising a transducer array including a plurality of ultrasound probes for transmitting and receiving ultrasound waves. The transducer array comprises preferably one or more 2D arrays of transducer elements for providing multi-dimensional image data.

The ultrasound imaging apparatus 10 comprises in general a processing unit 16 connected to the ultrasound acquisition unit 14 for evaluating the ultrasound data received from the ultrasound acquisition unit and for providing ultrasound images on the basis of the ultrasound data.

The ultrasound acquisition unit 14 is formed as a multi-probe array for transthoracic echocardiography (TTE) or as a large area transthoracic echocardiography (LATTE), which comprises the plurality of ultrasound probes which are preferably connected in a fixed relation to each other. In an alternative embodiment, the different ultrasound probes are movable with respect to each other so that these ultrasound probes can be utilized for providing ultrasound data individually from different viewing directions.

The processing unit 16 comprises a control unit 18 which is provided to control the ultrasound acquisition unit 14 and the ultrasound probes, receives the ultrasound data and controls steering directions of ultrasound beams originating from the ultrasound probes in order to adjust a focal point of the ultrasound probes. The beam steering may be realized as an electrical steering of two dimensional ultrasound array or a mechanical steering of one dimensional array. The processing unit 16 further comprises a detection unit 20, which is connected to the control unit 18 for receiving the ultrasound data of the ultrasound acquisition unit 14 and the different ultrasound probes. The detection unit 20 detects on the basis of the ultrasound data anatomical objects of the patient 12 in a field of view of the ultrasound acquisition unit 14 and/or the ultrasound probes and determines a spatial relationship of the anatomical object in the field of view with respect to each of the ultrasound probes. The spatial relationship is in particular an orientation of a viewing direction of each of the probes (the direction of the steered beams origination from each probe) to a surface of the anatomical object in the field of view which is called a viewing angle. The detection unit 20 comprises a segmentation unit for segmenting the ultrasound data and for providing segmentation data as a three-dimensional representation of the anatomical object in the field of view. On the basis of the so-determined segmentation data which can be formed of a mesh of triangles, the viewing angle can be precisely determined with low technical effort. The segmentation unit of the detection unit 20 is connected to an external or an internal database 22 for receiving a segmentation model of the anatomical object so that the segmentation data can be based on the predefined segmentation model and can be adapted to the ultrasound data received from the ultrasound acquisition unit 14.

The detection unit 20 is connected to a selection unit 24 which determines on the basis of the spatial relationship of the anatomical object and each of the ultrasound probes and in particular on the basis of at least one anatomical feature of the anatomical object a acquisition quality of one physical parameter which is detectable from the ultrasound data. Since the viewing angle of each of the ultrasound probes has a major influence on the detectability of structures or elements or fluids in the field of view, the expected signal strength quality of the physical parameter which is detectable in the field of view can be determined. On the basis of this acquisition quality or quality factor which is determined for each of the ultrasound probes, one or more of the ultrasound probes are selected by the selection unit 24 providing a high quality ultrasound measurement.

The processing unit 16 further comprises an evaluation unit 26 connected to the ultrasound acquisition unit 14 and to the selection unit 24 which evaluates the ultrasound data of the at least one selected ultrasound probe and determines the at least one physical parameter on the basis of the ultrasound data received from the at least one selected ultrasound probe. The physical parameter corresponds to an anatomical structure in the field of view or a motion of an anatomical structure or a fluid in the field of view in order to analyze a vital function of the patient 12. The evaluation unit 26 preferably provides image data on the basis of the ultrasound data and provides the image data to a display unit 28 for displaying the respective ultrasound image.

In use of the ultrasound imaging apparatus 10, at least one of the ultrasound probes acquires first an overview image and the detection unit 20 detects an anatomical object to be analyzed in the field of view. If the relative position of the ultrasound probes to each other is not known, each of the ultrasound probes acquires an overview image and the relative position of the ultrasound probes to each other is determines by the segmentation unit. The overview images are segmented using the model-based segmentation so that an anatomical object can be segmented even if parts of the anatomical object are obscured or cannot be detected.

From the relative position of the ultrasound probes to the anatomical object, the viewing angles of each of the ultrasound probes can be determined so that the expected image quality for each of the ultrasound probes and the respective viewing directions can be determined.

On the basis of the so determined expected image quality, the ultrasound probe providing the best image quality can be selected.

The segmentation model on the basis of which the segmentation data is determined may also comprise anatomical information of the anatomical object in particular a fiber direction of the anatomical object so that also a viewing angle with respect to the fiber direction can be determined and utilized to estimate or determine the expected image quality.

On the basis of the so determined image quality, the selection unit 24 can select for every situation which probe provides the best image quality. If a close-up of an anatomical structure should be acquired, the ultrasound probe with the smallest viewing angle is selected. If a composite (compound) image should be acquired, the setup of different probes is chosen which has the smallest average angle for all relevant anatomical structures of the anatomical object. In this case, the ultrasound data received from different ultrasound probes can be combined to the composite image e.g. by weighting the different ultrasound data.

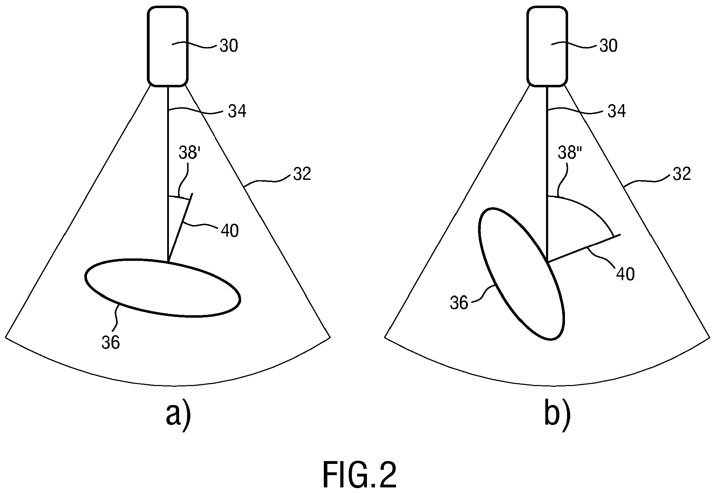

Fig. 2 shows a schematic illustration of different viewing angles dependent on a spatial relationship of the anatomical object to a viewing direction of an ultrasound probe. In Fig. 2 an ultrasound probe is schematically shown and generally denoted by 30. The ultrasound probe 30 emits and receives ultrasound waves (beams) in a field of view 32 and in a viewing direction 34. In the field of view 32 an anatomical object 36 is schematically shown and represented by a mesh of a segmentation model. A viewing angle 38 is shown as an angle between the viewing direction 34 and a normal direction 40 of a surface of the anatomical object 36. In Fig. 2a, the viewing angle 38' is relatively small (below 30° for example) and in Fig. 2b the viewing angle 38" is relatively large (beyond 45° or closer to 90°, for example).

The signal strength determined by the reflected ultrasound waves and, therefore, the image quality of the ultrasound image depends on the viewing angle 38 between the propagation direction of the ultrasound waves and the surface of the imaged anatomical object 36, wherein the signal strength is relatively low if the surface and the ultrasound waves are parallel to each other and the signal strength is relatively high if the surface and the ultrasound waves are almost perpendicular to each other such that the anatomical structure can be imaged with relatively high quality. Hence, if the viewing angle 38' is small as shown in Fig. 2a, the image quality is relatively high compared to the situation wherein the viewing angle 38" is close to 90°.

Hence, the expected image quality can be determined on the basis of the orientation of the surface or the fiber structure of the anatomical object 36 with respect to the viewing direction 34 of the ultrasound probe 30.

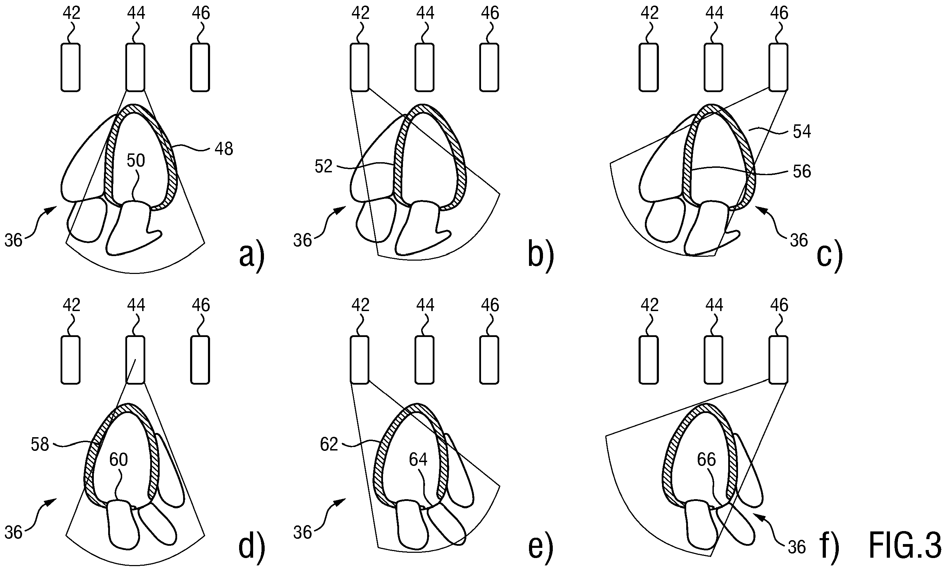

Fig. 3 shows the anatomical object 36 in different viewing directions of three ultrasound probes 42, 44, 46 of the ultrasound acquisition unit 14.

In Figs. 3a-c, the anatomical object which is a human heart is shown in one position, wherein the ultrasound probes 42, 44, 46 are disposed in different spatial relations to the anatomical object 36 so that each of the ultrasound probes 42, 44, 46 have different viewing angles 38 to a surface or a fiber structure of the anatomical object 36. In Fig. 3a, the ultrasound scan of the ultrasound probe 44 is shown, wherein the viewing angle 38 to a side surface of the anatomical object 30 shown at 48 is approximately 90° so that a low image quality can be expected. At a further position shown at 50, the viewing angle is low so that a high quality of the image can be expected at this position.

In Fig. 3b, the ultrasound scan of the ultrasound probe 42 is shown having a small viewing angle 38 to a side surface of the anatomical object 36 shown at 52 so that the image quality of this position is expected to be large. In Fig. 3c, the ultrasound scan of ultrasound probe 46 is shown, wherein the viewing angle 38 is small with respect to two surfaces shown at 54, 56 of the anatomical object 36 so that the image quality for these positions 54, 56 is expected to be high. Hence, the ultrasound image quality of the different positions of the anatomical object is different for the different viewing directions of the ultrasound probes 42, 44, 46. In order to form an ultrasound image one of the ultrasound probes 42, 44, 46 is selected for providing the image data or the ultrasound image is formed as a composite image of a plurality of the ultrasound probes 42, 44, 46 having the smallest viewing angles 38 for the relevant structures and can be weighted by a weight factor.

In Figs. 3d-f the anatomical object 36 is shown in a further orientation, wherein the three ultrasound probes 42, 44, 46 have different viewing angles to different structures of the anatomical object 36. In Fig. 3d, the ultrasound probe 44 is shown having a large viewing angle with respect to a side surface shown at 58 and a small viewing angle 38 for an inner structure shown at 60 so that the image quality of these structures is different. In Fig. 3e, the ultrasound probe 42 is shown having a small viewing angle with respect to two different structures shown at 62, 64 of the anatomical object 36 so that the image quality for these structures is expected to be high. In Fig. 3f, the ultrasound probe 46 is shown having a large viewing angle with respect to a structure of the anatomical object 36 shown at 66 so that the image quality for this structure is expected to be low. The ultrasound image is based upon the ultrasound data received from one or more selected ultrasound probes 42, 44, 46 providing the best image quality with respect to a relevant structure to be imaged. The ultrasound image may be based on a plurality of ultrasound data as a composite image, wherein the different data may be weighted dependent on the expected image quality.

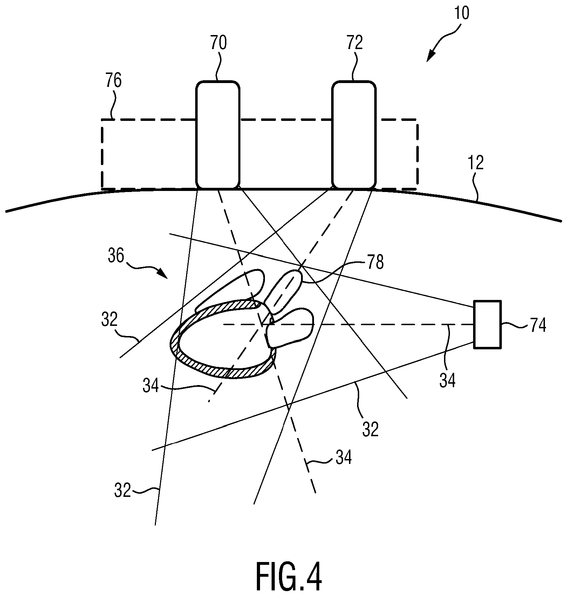

In Fig. 4 an embodiment of the ultrasound acquisition unit 14 is shown having a plurality of ultrasound probes 70, 72, 74, wherein the ultrasound probes 70, 72 are part of an ultrasound array 76 disposed at a skin of the patient 12 and the ultrasound probe 74 is inserted into the patient's body and formed as a transesophageal echocardiography (TEE) probe. The ultrasound probes 70, 72, 74 are in this embodiment utilized to measure a blood flow in the anatomical object 36 on the basis of a Doppler signal. To measure the motion of an anatomical structure or a fluid on the basis of the Doppler signal, the viewing direction and the motion vector should be parallel to provide a large signal strength and a high quality measurement. The direction of flow of the blood can be estimated on the basis of the detected anatomical object 36 and on the basis of the segmentation data so that one of the ultrasound probes 70, 72, 74 can be selected having the best viewing angle with respect to the direction of flow in order to receive a strong Doppler signal. In this case the viewing direction of the ultrasound probe 72 is parallel to the flow direction of the blood in a certain anatomical structure 78 of the anatomical object 36 so that this ultrasound probe 72 is selected for measuring the flow of the blood in this anatomical structure 78.

The flow direction may also be determined on the basis of the segmentation model, in which the flow directions can be stored as an anatomical feature of the anatomical object 36.

Hence, also the ultrasound probes 70, 72, 74 can be selected having the best viewing angle in order to receive a Doppler signal from the anatomical object 36.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Ultrasound imaging apparatus (10) comprising:
- an ultrasound acquisition unit (10) connected to a plurality of ultrasound probes (42, 44, 46, 70, 72, 74) each for providing ultrasound data suitable for ultrasound imaging of a patient (12) in a field of view (32) of the ultrasound probes,
- a detection unit (20) for detecting an anatomical object (36) of the patient in the field of view on the basis of the ultrasound data received from at least one of the ultrasound probes and for determining a spatial relationship of the anatomical object and each of the ultrasound probes,
- a selection unit (24) coupled to the detection unit for selecting at least one of the ultrasound probes on the basis of an acquisition quality of at least one physical parameter detectable from the ultrasound data, wherein the acquisition quality is determined on the basis of the spatial relationship (38) of at least one anatomical feature of the anatomical object and each of the ultrasound probes, and
- an evaluation unit (26) coupled to the ultrasound acquisition unit for receiving the ultrasound data from the at least one selected ultrasound probe and for determining the at least one physical parameter on the basis of the ultrasound data received from the selected ultrasound probe,
wherein the detection unit is adapted to detect as anatomical feature of the anatomical object (i) a fiber direction of the anatomical object, (ii) a surface of the anatomical object, or (iii) a flow direction of a fluid associated to the anatomical object, and wherein the detection unit is adapted to determine an orientation of (i) the fiber direction, (ii) the surface, or (iii) the flow direction with respect to a viewing direction (34) of each of the ultrasound probes as the spatial relationship.

2. Ultrasound imaging apparatus as claimed in claim 1, wherein the acquisition quality is relatively high for an orthogonal orientation of the surface or the fiber direction with respect to the viewing direction of the respective ultrasound probe.

3. Ultrasound imaging apparatus as claimed in claim 1, wherein the ultrasound probes are fixed to each other.

4. Ultrasound imaging apparatus as claimed in claim 1, wherein the detection unit comprises a segmentation unit for determining segmentation data of the anatomical object on the basis of the ultrasound data and for determining the spatial relationship on the basis of the segmentation data.

5. Ultrasound imaging apparatus as claimed in claim 1, wherein the detection unit is adapted to determine a spatial relationship of the ultrasound probes to each other on the basis of the spatial relationship of each of the probes to the anatomical object.

6. Ultrasound imaging apparatus as claimed in claim 1, wherein the physical parameter is an ultrasound image of the anatomical object.

7. Ultrasound imaging apparatus as claimed in claim 1, wherein the ultrasound acquisition unit comprises a control unit (18) for controlling a steering direction of ultrasound beams originating from the ultrasound probes on the basis of the spatial relationship to the anatomical object.

8. Ultrasound imaging apparatus as claimed in claim 1 further comprising an imaging unit for providing a compound ultrasound image on the basis of the ultrasound data received from the plurality of ultrasound probes.

9. Ultrasound imaging apparatus as claimed in claim 8, wherein the image data of each of the ultrasound probes is weighted on the basis of the determined acquisition quality.

10. Ultrasound imaging apparatus as claimed in claim 1, wherein the physical parameter is a flow parameter of the fluid.

11. Ultrasound imaging apparatus as claimed in claim 1, wherein the acquisition quality is relatively large for a parallel orientation of the flow direction with respect to a viewing direction of the respective ultrasound probe.

12. Ultrasound imaging method comprising the steps of:
- acquiring ultrasound data suitable for ultrasound imaging of a patient (12) in a field of view (32) of a plurality of ultrasound probes (42, 44, 46, 70, 72, 74),
- determining an anatomical object (36) of the patient in the field of view on the basis of the ultrasound data received from at least one of the ultrasound probes,
- detecting as anatomical feature of the anatomical object (i) a fiber direction of the anatomical object, (ii) a surface of the anatomical object, or (iii) a flow direction of a fluid associated to the anatomical object;
- determining a spatial relationship of the anatomical object and each of the ultrasound probes, wherein the spatial relationship includes an orientation of (i) the fiber direction, (ii) the surface, or (iii) the flow direction with respect to a viewing direction (34) of each of the ultrasound probes,
- selecting at least one of the ultrasound probes on the basis of an acquisition quality of at least one physical parameter detectable from the ultrasound data, wherein the acquisition quality is determined on the basis of the spatial relationship of at least one anatomical feature of the anatomical object and each of the ultrasound probes, and
- determining the at least one physical parameter on the basis of the ultrasound data received from the selected ultrasound probe.

13. Computer program comprising program code means for causing a computer to carry out the steps of the method as claimed in claim 12, when said computer program is carried out on a computer.

## Patentansprüche

1. Ultraschall-Bildgebungsgerät (10), das Folgendes umfasst:
- eine Ultraschallerfassungseinheit (10), die mit mehreren Ultraschallsonden (42, 44, 46, 70, 72, 74) verbunden ist, die für die Ultraschallbildgebung für einen Patienten (12) jeweils die Ultraschalldaten im Bildfeld (32) der Ultraschallsonden bereitstellen,
- eine Erkennungseinheit (20) zum Erkennen eines anatomischen Objekts (36) des Patienten im Bildfeld anhand der von mindestens einer der Ultraschallsonden abgerufenen Ultraschalldaten sowie zum Ermitteln der räumlichen Beziehung zwischen dem anatomischen Objekt und den einzelnen Ultraschallsonden,
- eine mit der Erfassungseinheit verbundene Auswahleinheit (24) zum Auswählen mindestens einer der Ultraschallsonden anhand der Erfassungsqualität mindestens eines der anhand der Ultraschalldaten erkennbaren physikalischen Parameter, wobei die Erfassungsqualität anhand der räumlichen Beziehung (38) mindestens eines anatomischen Merkmals des anatomischen Objekts zu den einzelnen Ultraschallsonden ermittelt wird, und
- eine mit der Ultraschallerfassungseinheit verbundene Auswertungseinheit (26) zum Abrufen der Ultraschalldaten von der mindestens einen ausgewählten Ultraschallsonde sowie zum Ermitteln des mindestens einen physikalischen Parameters anhand der von der ausgewählten Ultraschallsonde abgerufenen Ultraschalldaten,
wobei die Erkennungseinheit Folgendes als anatomisches Merkmal des anatomischen Objekts erkennt: (i) eine Faserrichtung des anatomischen Objekts; (ii) eine Oberfläche des anatomischen Objekts, oder (iii) eine Strömungsrichtung einer dem anatomischen Objekt zugeordneten Flüssigkeit, und wobei die Erkennungseinheit die Ausrichtung (i) der Faserrichtung, (ii) der Oberfläche oder (iii) der Strömungsrichtung in Bezug auf die Blickrichtung (34) der einzelnen Ultraschallsonden als räumliche Beziehung ermittelt.

2. Ultraschall-Bildgebungsgerät gemäß Anspruch 1, wobei die Erfassungsqualität für die orthogonale Ausrichtung der Oberfläche oder Faserrichtung in Bezug auf die Blickrichtung der entsprechenden Ultraschallsonde relativ hoch ist.

3. Ultraschall-Bildgebungsgerät gemäß Anspruch 1, wobei die Ultraschallsonden miteinander verbunden sind.

4. Ultraschall-Bildgebungsgerät gemäß Anspruch 1, wobei die Erkennungseinheit eine Segmentierungseinheit zum Ermitteln von Segmentierungsdaten für das anatomische Objekt anhand der Ultraschalldaten sowie zum Ermitteln der räumlichen Beziehung anhand der Segmentierungsdaten umfasst.

5. Ultraschall-Bildgebungsgerät gemäß Anspruch 1, wobei die Erkennungseinheit anhand der räumlichen Beziehung der einzelnen Sonden zum anatomischen Objekt eine räumliche Beziehung der Ultraschallsonden zueinander ermittelt.

6. Ultraschall-Bildgebungsgerät gemäß Anspruch 1, wobei es sich beim physikalischen Parameter um ein Ultraschallbild des anatomischen Objekts handelt.

7. Ultraschall-Bildgebungsgerät gemäß Anspruch 1, wobei die Ultraschallerfassungseinheit eine Steuerungseinheit (18) zum Steuern der Lenkrichtung der von den Ultraschallsonden ausgehenden Ultraschallstrahlen anhand der räumlichen Beziehung zum anatomischen Objekt umfasst.

8. Ultraschall-Bildgebungsgerät gemäß Anspruch 1, das zudem eine Bildgebungseinheit zum Bereitstellen eines zusammengeführten Ultraschallbilds anhand der von den Ultraschallsonden abgerufenen Ultraschalldaten umfasst.

9. Ultraschall-Bildgebungsgerät gemäß Anspruch 8, wobei die Bilddaten der einzelnen Ultraschallsonden anhand der ermittelten Erfassungsqualität gewichtet werden.

10. Ultraschall-Bildgebungsgerät gemäß Anspruch 1, wobei es sich beim physikalischen Parameter um einen Strömungsparameter der Flüssigkeit handelt.

11. Ultraschall-Bildgebungsgerät gemäß Anspruch 1, wobei die Erfassungsqualität für die parallele Ausrichtung der Strömungsrichtung in Bezug auf die Blickrichtung der entsprechenden Ultraschallsonde relativ hoch ist.

12. Ultraschall-Bildgebungsmethode, die folgende Schritte umfasst:
- Erfassen von Ultraschalldaten für die Ultraschallbildgebung eines Patienten (12) im Blickfeld (32) mehrerer Ultraschallsonden (42, 44, 46, 70, 72, 74),
- Ermitteln eines anatomischen Objekts (36) des Patienten im Blickfeld anhand der von mindestens einer der Ultraschallsonden empfangenen Ultraschalldaten,
- Erfassen der folgenden Aspekte als anatomisches Merkmal des anatomischen Objekts: (i) eine Faserrichtung des anatomischen Objekts; (ii) eine Oberfläche des anatomischen Objekts, oder (iii) eine Strömungsrichtung einer dem anatomischen Objekt zugeordneten Flüssigkeit;
- Ermitteln der räumlichen Beziehung des anatomischen Objekts zu den einzelnen Ultraschallsonden, wobei die räumliche Beziehung die Ausrichtung (i) der Faserrichtung, (ii) der Oberfläche oder (iii) der Strömungsrichtung in Bezug auf die Blickrichtung (34) der einzelnen Ultraschallsonden umfasst,
- Auswählen mindestens einer der Ultraschallsonden anhand der Erfassungsqualität mindestens eines der anhand der Ultraschalldaten erkennbaren physikalischen Parameter, wobei die Erfassungsqualität anhand der räumlichen Beziehung mindestens eines anatomischen Merkmals des anatomischen Objekts zu den einzelnen Ultraschallsonden ermittelt wird,
und
- Ermitteln des mindestens einen physikalischen Parameters anhand der von der ausgewählten Ultraschallsonde abgerufenen Ultraschalldaten.

13. Ein Computerprogramm, das Programmcode umfasst, mit dem ein Computer veranlasst wird, die Schritte der Methode gemäß Anspruch 12 auszuführen, wenn das Computerprogramm auf einem Computer ausgeführt wird.

## Revendications

1. Appareil d'imagerie par ultrasons (10) comprenant :
- une unité d'acquisition d'ultrasons (10) connectée à une pluralité de sondes ultrasonores (42, 44, 46, 70, 72, 74) chacune destinée à la fourniture des données ultrasonores appropriées pour l'imagerie par ultrasons d'un patient (12) dans un champ de vision (32) des sondes ultrasonores,
- une unité de détection (20) destinée à la détection d'un objet anatomique (36) du patient dans le champ de vision en fonction des données ultrasonores reçues de l'au moins une des sondes ultrasonores et à la détection d'une relation spatiale de l'objet anatomique et chacune des sondes ultrasonores,
- une unité de sélection (24) couplée à l'unité de détection pour sélectionner l'au moins une des sondes ultrasonores en fonction d'une qualité d'acquisition d'au moins un paramètre physique détectable à partir des données ultrasonores, dans lequel la qualité d'acquisition est déterminée en fonction de la relation spatiale (38) d'au moins une caractéristique anatomique de l'objet anatomique et de chacune des sondes ultrasonores, et
- une unité d'évaluation (26) couplée à l'unité d'acquisition d'ultrasons pour recevoir les données ultrasonores de l'au moins une sonde ultrasonore sélectionnée et pour déterminer l'au moins un paramètre physique en fonction des données ultrasonores reçues de la sonde ultrasonore sélectionnée,
dans lequel l'unité de détection est conçue pour détecter en tant que caractéristique anatomique de l'objet anatomique (i) une direction de fibre de l'objet anatomique, (ii) une surface de l'objet anatomique, ou (iii) une direction d'écoulement d'un fluide associé à l'objet anatomique, et dans lequel l'unité de détection est conçue pour déterminer une orientation (i) de la direction de la fibre, (ii) de la surface, ou (iii) de la direction d'écoulement par rapport à une direction d'observation (34) de chacune des sondes ultrasonores en tant que relation spatiale.

2. Appareil d'imagerie par ultrasons selon la revendication 1, dans lequel la qualité d'acquisition est relativement élevée pour une orientation orthogonale de la surface ou de la direction de la fibre par rapport à la direction d'observation de la sonde ultrasonore respective.

3. Appareil d'imagerie par ultrasons selon la revendication 1, dans lequel les sondes ultrasonores sont fixées les unes aux autres.

4. Appareil d'imagerie par ultrasons selon la revendication 1, dans lequel l'unité de détection comprend une unité de segmentation destinée à la détermination des données de segmentation de l'objet anatomique en fonction des données ultrasonores et à la détermination de la relation spatiale en fonction des données de segmentation.

5. Appareil d'imagerie par ultrasons selon la revendication 1, dans lequel l'unité de détection est conçue pour déterminer une relation spatiale des sondes ultrasonores les unes par rapport aux autres en fonction de la relation spatiale de chacune des sondes avec l'objet anatomique.

6. Appareil d'imagerie par ultrasons selon la revendication 1, dans lequel le paramètre physique est une image ultrasonore de l'objet anatomique.

7. Appareil d'imagerie par ultrasons selon la revendication 1, dans lequel l'unité d'acquisition d'ultrasons comprend une unité de commande (18) destinée à la commande d'un sens de direction de faisceaux ultrasonores provenant des sondes ultrasonores en fonction de la relation spatiale avec l'objet anatomique.

8. Appareil d'imagerie par ultrasons selon la revendication 1, comprenant en outre une unité d'imagerie destinée à la fourniture d'une image ultrasonore composée en fonction des données ultrasonores reçues de la pluralité de sondes ultrasonores.

9. Appareil d'imagerie par ultrasons selon la revendication 8, dans lequel les données d'image de chacune des sondes ultrasonores sont pondérées en fonction de la qualité d'acquisition déterminée.

10. Appareil d'imagerie par ultrasons selon la revendication 1, dans lequel le paramètre physique est un paramètre d'écoulement du fluide.

11. Appareil d'imagerie par ultrasons selon la revendication 1, dans lequel la qualité d'acquisition est relativement large pour une orientation parallèle de la direction d'écoulement par rapport à une direction d'observation de la sonde ultrasonore respective.

12. Procédé d'imagerie par ultrasons comprenant les étapes suivantes :
- l'acquisition des données ultrasonores appropriées pour l'imagerie par ultrasons d'un patient (12) dans un champ de vision (32) d'une pluralité de sondes ultrasonores (42, 44, 46, 70, 72, 74),
- la détermination d'un objet anatomique (36) du patient dans le champ de vision en fonction des données ultrasonores reçues de l'au moins une des sondes ultrasonores,
- la détection en tant que caractéristique anatomique de l'objet anatomique (i) d'une direction de fibre de l'objet anatomique, (ii) d'une surface de l'objet anatomique, ou (iii) d'une direction d'écoulement d'un fluide associé à l'objet anatomique,
- la détermination d'une relation spatiale entre l'objet anatomique et chacune des sondes échogra-phiques, dans lequel la relation spatiale comprend une orientation (i) de la direction des fibres, (ii) de la surface, ou (iii) de la direction d'écoulement par rapport à une direction d'observation (34) de chacune des sondes ultrasonores,
- la sélection de l'au moins une des sondes ultrasonores en fonction d'une qualité d'acquisition d'au moins un paramètre physique détectable à partir des données ultrasonores, dans lequel la qualité d'acquisition est déterminée en fonction de la relation spatiale de l'au moins une caractéristique anatomique de l'objet anatomique et de chacune des sondes ultrasonores,
et
- la détermination de l'au moins un paramètre physique en fonction des données ultrasonores reçues de la sonde ultrasonore sélectionnée.

13. Programme informatique comprenant un moyen de code de programme permettant d'amener un ordinateur à exécuter les étapes du procédé selon la revendication 12, lorsque ledit programme informatique est exécuté sur un ordinateur.
